# EUROPÄISCHE PATENTSCHRIFT

(11) **EP 3 188 767 B1**
(45) Veröffentlichungstag und Bekanntmachung des Hinweises auf die Patenterteilung: **16.01.2019**
(21) Anmeldenummer: 15757236.3
(22) Anmeldetag: 28.08.2015
(51) Int. Cl.: A61M 1/00, A61G 7/05, A61G 13/10, G01F 22/00

(54) **AUFHÄNGEVORRICHTUNG EINES DRAINAGEBEHÄLTERS**
SUSPENSION ASSEMBLY OF A DRAINAGE CONTAINER
DISPOSITIF DE SUSPENSION D'UN RÉCIPIENT DE DRAINAGE

(30) Priorität: 02.09.2014 EP 14183201
(43) Veröffentlichungstag der Anmeldung: 12.07.2017
(73) Patentinhaber: Medela Holding AG, 6340 Baar (CH)
(72) Erfinder: GIEZENDANNER, Charles, CH-6443 Morschach (CH); BÄCHLER, Cornel, CH-6038 Gisikon (CH); EHLERT, Hilmar, CH-6052 Hergiswil (CH)
(74) Vertreter: Rutz, Andrea
(86) Internationale Anmeldenummer: PCT/EP2015/069755
(87) Internationale Veröffentlichungsnummer: WO 2016/034510

(56) Entgegenhaltungen:
- WO-A1-97/10856
- WO-A2-94/21311
- DE-U1- 20 217 847
- US-A- 5 756 940
- US-A1- 2007 106 177
- US-A1- 2013 165 877

## Beschreibung

### TECHNISCHES GEBIET

Die vorliegende Erfindung betrifft eine Aufhängevorrichtung eines Drainagebehälters, eine Befestigungseinrichtung einer derartigen Aufhängevorrichtung, ein Befestigungsteil einer derartigen Aufhängevorrichtung sowie ein Drainagesystem mit einer derartigen Aufhängevorrichtung.

### STAND DER TECHNIK

Drainagebehälter, auch Fluidsammelbehälter genannt, sind Auffangbehälter für Körperflüssigkeiten, welche beim Absaugen im Rahmen medizinischer und kosmetischer Behandlungen anfallen. Die Körperflüssigkeit wird mittels einer Vakuumpumpe über eine patientenseitige Drainageleitung oder Saugleitung in den Behälter geleitet. Der Behälter weist hierfür einen Anschluss für die patientenseitige Drainageleitung und einen Anschluss für die Saug- oder Vakuumpumpe oder für ein Zentralvakuum des Spitals auf. Der Drainagebehälter kann ein Einmalgebrauch-Behälter sein oder mindestens Teile davon können mehrfach verwendet werden.

WO 2007/085100 und WO 2013/177716 offenbaren Drainagebehälter mit einem Aussenbehälter, einem diesen Aussenbehälter dichtend verschliessenden Deckel und einem Beutel, welcher im Aussenbehälter aufgenommen ist und dicht mit dem Deckel verbunden ist. Deckel und Beutel lassen sich gemeinsam vom Aussenbehälter entfernen. Diese Behälter lassen sich mittels einer Schnappverbindung mit einer Aufhängevorrichtung verbinden, welche wiederum an einer Schiene befestigbar ist. Die Schiene ist vorzugsweise eine Wandschiene des Spitals oder der Pflegestation oder eine Bettschiene. Diese Aufhängevorrichtung weist einen Grundkörper auf, welcher auf einer Seite mit einer federbelasteten Klammer zum Einklemmen der Schiene und auf der gegenüberliegenden Seite mit einer vertikal verlaufenden Nut versehen ist. Der Aussenbehälter weist einen entsprechenden in vertikaler Richtung verlaufenden Nutstein auf, welcher in die Nut einschiebbar ist. Der Deckel mit dem Beutel ist für den einmaligen Gebrauch bestimmt und wird anschliessend entsorgt. Der Drainagebehälter lässt sich mehrfach verwenden, jedoch nur begrenzt. Die Aufhängevorrichtung hingegen wird über mehrere Jahre hinweg gebraucht und auch zur Befestigung von anderen medizinischen Vorrichtungen verwendet. Diese Drainagebehälter sowie diese Aufhängevorrichtungen haben sich in der Praxis bewährt.

Der Füllstand der Drainagebehälter muss regelmässig kontrolliert werden, da er einerseits einen Hinweis auf den Drainageerfolg gibt und andererseits nicht überfüllt werden darf. Diese Kontrolle erfolgt im Stand der Technik visuell durch das Pflegepersonal. Dies ist insbesondere dann erschwert oder verunmöglicht, wenn die Flüssigkeit nicht direkt in einem starren Behälter mit Füllstandsanzeige, sondern in einem Beutel gesammelt wird. Es besteht somit das Bedürfnis, derartige Drainagebehälter mit einer Vorrichtung zur Messung der Füllmenge zu versehen.

In artfremden Gebieten sind Füllmengenbestimmungen bekannt. So beschreibt CN 202599512 U einen Becher mit einem Handgriff, in welchen eine Tasse eingehängt werden kann. Im Handgriff ist ein Wiegesensor vorhanden, um das Gewicht der Tasse und deren Inhalt zu bestimmen.

CN 2018997492 U zeigt einen Krug mit einem fest damit verbundenen Handgriff, welcher wiederum einen Wiegesensor aufweist.

In CN 202158898 U ist eine ringförmige Bodenplatte mit einer Wiegevorrichtung vorhanden, an welcher ein Handgriff angeformt ist. Auf diese Bodenplatte lässt sich ein Krug stellen.

US 2010/0089152 und DE 20 2010 010 088 U offenbaren einen Krug mit Handgriff und einer Bodenplatte mit Wiegevorrichtung.

In US 374 759 und US 2 478 272 werden bewegbare mechanische Anzeigen verwendet, um die Füllmenge eines Behälters anzuzeigen.

In WO 97/10856 wird die Anordnung einer Wägezelle zwischen einem Ständer und einem daran angehängten Behälter vorgeschlagen, um das Gewicht des Behälters zu messen.

DE 202 17 847 offenbart eine Aufhäng- und Wägeeinrichtung, um einen Sekretsammelbeutel an einem Gehäuse einer Sekretsammelvorrichtung einzuhängen.

US 2013/0165877 zeigt die Aufhängung eines Drainagebehälters an einem vertikalen Ständer via ein dazwischen angeordnetes Wägeelement.

In US 2007/0106177 ist ein Sammelbeutel offenbart, der an einem Ende eines langestreckten Trägers angehängt ist. Der langgestreckte Träger weist eine Wägezelle auf.

WO 94/21311 offenbart eine an einer vertikalen Stange befestigbare Aufhäng- und Wägevorrichtung mit einer ringförmigen Klammer zum Halten eines Drainagebehälters.

US 5,756,940 zeigt einen an einer Wägeeinrichtung aufgehängten Sammelbehälter. Die Wägeeinrichtung ist ihrerseits mit einer vertikalen Stange verbunden.

### DARSTELLUNG DER ERFINDUNG

Es ist eine Aufgabe der Erfindung, die Bestimmung einer Füllmenge eines Drainagebehälters zu erleichtern.

Diese Aufgabe löst eine Aufhängevorrichtung mit den Merkmalen des Patentanspruchs 1.

Die erfindungsgemässe Aufhängevorrichtung eines Drainagebehälters, welcher zum Auffangen von abgesaugter Körperflüssigkeit dient, weist auf einer ersten Seite eine erste Befestigungseinheit zum Anhängen und Befestigen des Drainagebehälters, vorzugsweise seines Aussenbehälters, an die Aufhängevorrichtung auf und auf einer zweiten Seite eine zweite Befestigungseinheit zum Anhängen und Befestigen der Aufhängevorrichtung an eine Schiene. Die erste Befestigungseinheit ist in einem ersten Befestigungsteil und die zweite Befestigungseinheit in einem zweiten Befestigungsteil angeordnet. Die Aufhängevorrichtung umfasst ein Wägeteil mit einem Wägeelement, wobei das Wägeteil mit dem ersten Befestigungsteil und dem zweiten Befestigungsteil verbunden ist oder verbindbar ist, um mit dem Wägeelement eine relative Verschiebung zwischen dem ersten und dem zweiten Befestigungsteil zu messen, so dass eine Füllmenge der abgesaugten Körperflüssigkeit im Drainagebehälter bestimmbar ist. Die zweite Seite ist vorzugsweise benachbart oder gegenüberliegend zur ersten Seite angeordnet.

Die Aufhängevorrichtung definiert eine Aufhängeachse zur Aufhängung des Drainagebehälters, wobei ein Neigungssensor vorhanden ist zur Feststellung eines Winkels zwischen der Aufhängeachse und einer Vertikalen im Raum, in welchem sich die Aufhängevorrichtung befindet. Dadurch lässt sich feststellen, ob die Schiene und somit der Drainagebehälter schief steht, und es lassen sich dadurch entstehende Messfehler elektronisch kompensieren. Alternativ oder zusätzlich kann auch gewarnt werden, wenn der Behälter zu schief hängt und umzukippen droht.

Dadurch, dass die Messung in der Aufhängevorrichtung und nicht im Behälter selber stattfindet, lässt sich auch die Füllmenge bzw. das Gewicht des Inhalts eines Beutels genau bestimmen.

Des Weiteren hat dies den Vorteil, dass zeitlich nur begrenzt einsetzbare Behälter nach wie vor kostengünstig hergestellt werden können und die elektronischen Teile in der wiederverwendbaren Aufhängevorrichtung integriert sind. Dies ist insbesondere dann der Fall, wenn die erste Befestigungseinheit zur Erstellung einer bei bestimmungsgemässem Gebrauch der Aufhängevorrichtung lösbaren Verbindung mit dem Drainagebehälter, vorzugsweise mit seinem Aussenbehälter, ausgebildet ist.

Vorzugsweise ist alternativ oder zusätzlich auch die zweite Befestigungseinheit zur Erstellung einer bei bestimmungsgemässem Gebrauch der Aufhängevorrichtung lösbaren Verbindung mit der Schiene ausgebildet.

In einer alternativen Ausführungsform sind jedoch die Aufhängevorrichtung oder mindestens das erste Befestigungsteil und das Wägeteil fest mit dem Drainagebehälter, insbesondere dem Aussenbehälter, verbunden.

In einer bevorzugten Ausführungsform bilden das erste Befestigungsteil und das Wägeteil eine fest miteinander verbundene und bei bestimmungsgemässem Gebrauch der Aufhängevorrichtung nicht zur Trennung voneinander vorgesehene Einheit.

Unter bestimmungsgemässem Gebrauch wird in diesem Text ein Gebrauch verstanden, welcher dazu dient, Körperflüssigkeit eines Patienten abzusaugen und welcher hierzu bedingt, dass der Drainagebehälter korrekt befestigt ist, um diese Aufgabe zu erfüllen. Die Erstellung bzw. die Lösung der Verbindungen kann dabei vor oder nach der Absaugung erfolgen. Eine Trennung während des Absaugens ist vorzugsweise ebenfalls möglich. Zur Erstellung und Trennung der Verbindung werden vorzugsweise keine Werkzeuge benötigt, sondern sie lässt sich vorzugsweise von Hand durchführen.

Vorzugsweise bildet das zweite Befestigungsteil eine bei bestimmungsgemässem Gebrauch der Aufhängevorrichtung lösbare Verbindung mit dem Wägeteil. Dies hat den Vorteil, dass das Befestigungsteil auch zur Befestigung von anderen Vorrichtungen verwendet werden kann. Insbesondere kann ein zweites Befestigungsteil verwendet werden, wie es beispielsweise in WO 2007/085100 beschrieben ist.

In einer anderen Ausführungsform bilden das zweite Befestigungsteil und das Wägeteil eine fest miteinander verbundene und bei Gebrauch nicht zur Trennung voneinander vorgesehene Einheit, wodurch das erste Befestigungsteil, das Wägeteil und das zweite Befestigungsteil eine bei Gebrauch nicht zur Trennung voneinander vorgesehene Einheit bilden. Dies hat die Vorteile, dass die Aufhängevorrichtung trotz des integrierten Wägeelements kompakt ausgebildet und relativ kostengünstig ist und dass auch einzelne Teile nicht verloren gehen können.

Als Wägeelement eignet sich insbesondere eine Wägezelle, ein Federelement mit Wegmessung, ein Piezosensor oder ein Drucksensor mit Linearführung.

In einer bevorzugten Ausführungsform weist das Wägeelement, insbesondere die Wägezelle, einen ersten Bereich auf, welcher fest mit dem ersten Befestigungsteil verbunden ist, wobei das Wägeelement einen zweiten Bereich aufweist, welcher fest mit dem Wägeteil verbunden ist. Dadurch lässt sich der zweite Befestigungsteil relativ beliebig ausbilden, und er lässt sich insbesondere mit einer im Gebrauch lösbaren Verbindung mit dem Wägeteil verbinden.

Vorzugsweise weist das zweite Befestigungsteil einen Überlastschutz auf. Dieser ist durch einen in vertikaler Richtung verschiebbaren federbelasteten Bolzen gebildet, auf welchem das Wägeteil aufliegt. Vorzugsweise oder alternativ weist das zweite Befestigungsteil einen Vorsprung auf, welcher sich bis zum ersten Befestigungsteil erstreckt und welcher bei einer Gewichtsüberlast zur Auflage des ersten Befestigungsteils dient. Diese Ausführungsformen ermöglichen einen Überlastschutz, welcher eine Überlastung des Wägeelements, insbesondere eine Überdehnung von Dehnungsmessstreifen der Wägezelle verhindert.

Um die Gewichtsbestimmung möglichst genau durchführen zu können, weist die Aufhängevorrichtung vorzugsweise eine Zugentlastung auf für einen mit dem Drainagebehälter verbundenen Drainageschlauch, welcher zum Absaugen der Körperflüssigkeit dient. Dieser Drainageschlauch wird vorzugsweise aufgehängt. Vorzugsweise weist die Zugentlastung einen Bügel auf, welcher vom Wägeteil und/oder vom zweiten Befestigungsteil getragen ist und welcher wiederum den Drainageschlauch trägt.

Vorzugsweise weist die Aufhängevorrichtung ein Display zur Anzeige der gemessenen Füllmenge auf, damit das Pflegepersonal, die medizinischen Fachpersonen oder auch die Patienten die Messwerte vor Ort ablesen können. Je nach Ausführungsform ist ein akustischer und/oder optischer Signalgeber vorhanden, welcher vor einer drohenden Überfüllung warnt.

In einer bevorzugten Ausführungsform weist die Aufhängevorrichtung eine Elektronikeinheit zur Übertragung der bestimmten Füllmenge und/oder eines Warnsignals an einen externen Empfänger auf. Dieser externe Empfänger kann die Vakuumpumpe, eine Steuerung des Zentralvakuums oder eine Ventileinheit zum Zentralvakuum sein. Vorzugsweise ist der externe Empfänger ein klinisches Netzwerk. Vorzugsweise werden die gemessenen Werte direkt in die Patientendaten eingetragen und/oder erzeugen ein Warnsignal im Stationsraum des Pflegepersonals.

Es lassen sich auch mehrere Drainagebehälter hintereinander oder nebeneinander schalten, wobei die Messwerte aller Drainagebehälter summiert und auf einem gemeinsamen Display vor Ort angezeigt werden. Die Signale lassen sich alternativ oder zusätzlich auch gemeinsam einem externen Empfänger zuführen.

Die gemessenen Werte lassen sich weiter verarbeiten. So lässt sich der Durchfluss bestimmen. Bei Absaugung an mehreren Stellen des Patienten, z.B. bei Fettabsaugung, lässt sich ein Vergleich zwischen den verschiedenen abgesaugten Flüssigkeitsmengen erstellen.

Teile der Aufhängevorrichtung lassen sich auch einzeln herstellen und vertreiben. Beispielsweise weist eine Befestigungseinrichtung der erfindungsgemässen Aufhängevorrichtung ein erstes Befestigungsteil mit einer ersten Befestigungseinheit zum Anhängen und Befestigen des Drainagebehälters an die Aufhängevorrichtung auf und umfasst ferner ein Wägeteil mit einem Wägeelement. Das Wägeteil ist mit dem ersten Befestigungsteil verbunden, und das Wägeteil ist mit einem zweiten Befestigungsteil zwecks Verbindung mit einer Schiene verbindbar, um mit dem Wägeelement eine relative Verschiebung zwischen dem ersten und zweiten Befestigungsteil zu messen, so dass eine Füllmenge der abgesaugten Körperflüssigkeit im Drainagebehälter bestimmbar ist.

Ferner lässt sich ein zweites Befestigungsteil der erfindungsgemässen Aufhängevorrichtung einzeln herstellen und vertreiben. Dieses zweite Befestigungsteil weist eine zweite Befestigungseinheit auf zur Verbindung der Aufhängevorrichtung an einer Schiene. Es besitzt einen Gewichtsüberlastschutz, wobei der Gewichtsüberlastschutz einen federbelasteten Bolzen zum Tragen des Wägeteils aufweist. Alternativ oder zusätzlich kann ein Vorsprung vorhanden sein, auf welchem das erste Befestigungsteil bei einer Überlastung aufliegt.

In einer bevorzugten Ausführungsform bildet die erfindungsgemässe Aufhängevorrichtung gemeinsam mit einem Drainagebehälter ein Drainagesystem. Der Drainagebehälter weist dabei einen Aussenbehälter, einen Beutel zur Aufnahme im Aussenbehälter und einen den Aussenbehälter dicht verschliessenden Deckel auf. Der Beutel ist dicht mit dem Deckel verbunden und ist gemeinsam mit dem Deckel vom Aussenbehälter entfernbar. Der Aussenbehälter weist ferner eine Befestigungseinheit zur Verbindung mit der ersten Befestigungseinheit des ersten Befestigungsteils auf. Die erfindungsgemässe Aufhängevorrichtung lässt sich jedoch auch mit anderen Arten von Drainagebehältern, insbesondere solchen ohne Beutel, verwenden.

Weitere Ausführungsformen sind in den abhängigen Ansprüchen angegeben.

### KURZE BESCHREIBUNG DER ZEICHNUNGEN

Eine bevorzugte Ausführungsform der Erfindung wird im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung eines erfindungsgemässen Drainagesystems;
- Figur 2: eine Seitenansicht des Drainagesystems gemäss Figur 1;
- Figur 3: eine perspektivische Darstellung eines Deckels mit einem Beutel des Drainagesystems gemäss Figur 1;
- Figur 4: eine Explosionsdarstellung einer Aufhängevorrichtung des Drainagesystems gemäss Figur 1;
- Figur 5: eine perspektivische Darstellung der Aufhängevorrichtung gemäss Figur 4 im zusammen gefügten Zustand;
- Figur 6: eine perspektivische Darstellung der Aufhängevorrichtung gemäss Figur 4 mit einer Zugentlastung und
- Figur 7: eine perspektivische Darstellung der zusammen gefügten Aufhängevorrichtung mit Display.

### BESCHREIBUNG BEVORZUGTER AUSFÜHRUNGSFORMEN

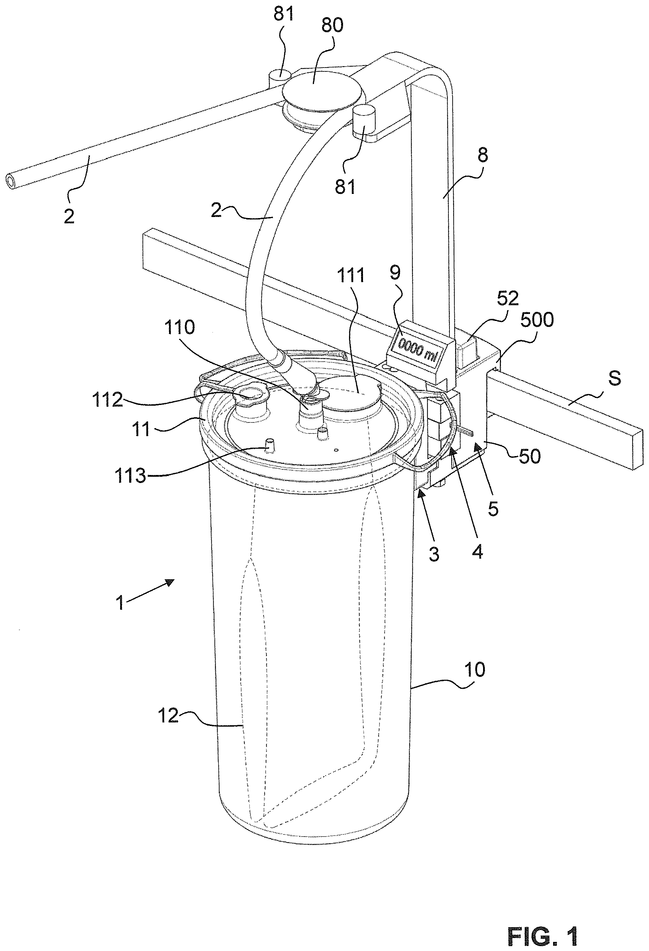

Figur 1 zeigt einen Drainagebehälter 1 mit einem starren, vorzugsweise durchsichtigen Aussenbehälter 10, einem diesen dicht verschliessenden Deckel 11 und einen am Deckel 11 dicht befestigten und im Aussenbehälter 10 aufgenommenen Beutel 12.

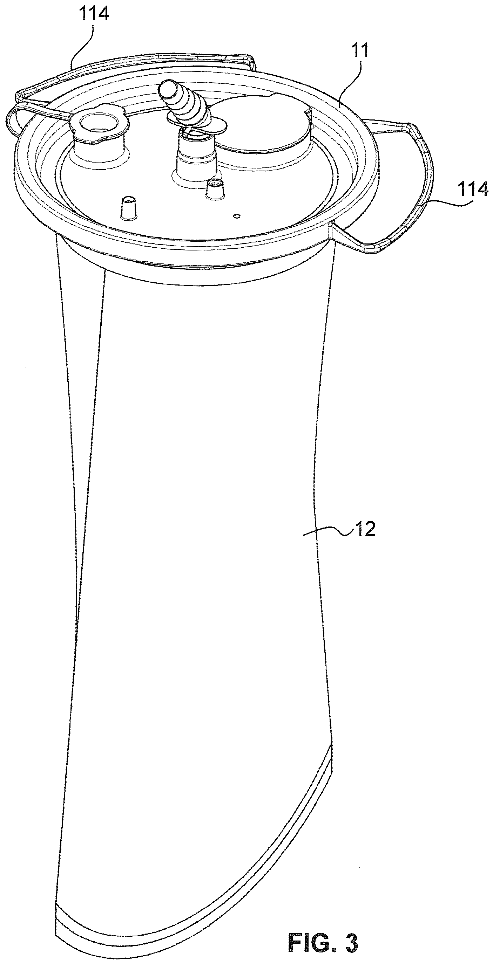

Der Behälter 1 weist die üblichen, für die Verbindung zum Patienten und zur Vakuumquelle notwendigen Mittel auf. Diese sind beispielsweise aus WO 2007/085100 und WO 2013/177716 hinlänglich bekannt. Am Deckel 11 ist ein Drainageanschluss 110 zur Verbindung des Beutelinnenraums mit einem Drainageschlauch 2 vorhanden, wobei der Drainageschlauch 2 zum Patienten führt und die abgesaugte Flüssigkeit in den Behälter leitet. Ferner weist der Deckel 11 vorzugsweise einen seriellen Anschluss 112 auf, damit er in Serie mit einem zweiten Drainagebehälter verbunden werden kann. Die Bezugsziffer 113 bezeichnet einen Befestigungsstutzen, auf welchem üblicherweise Verschlüsse bei Nichtgebrauch aufgesteckt werden. Die Bezugsziffer 111 bezeichnet eine Filterabdeckung, welche ebenfalls zum Beutelinnenraum führt, jedoch einen weiteren Kanal aufweist, der im Zwischenraum zwischen Aussenbehälter und Beutel endet und welcher eine Verbindung zu einem seitlichen Sauganschluss im Aussenbehälter 10 schafft. Der Sauganschluss dient zur Verbindung mit der Vakuumquelle. Er ist hier nicht sichtbar, jedoch aus WO 2013/177716 bekannt. Handgriffe 114 erleichtern das Entfernen des Deckels 11 und des Beutels 12 vom Aussenbehälter 10. Der Deckel 11 mit dem daran befestigten, vorzugsweise angeklebten oder mit ihm verschweissten, Beutel 12 ist in Figur 3 dargestellt.

Der Drainagebehälter 1, genauer der Aussenbehälter 10, ist seitlich an einer Aufhängevorrichtung 3, 4, 5 aufgehängt und befestigt. Die Aufhängevorrichtung 3, 4, 5 lässt sich an eine Schiene S, z.B. an eine Wandschiene oder an eine Bettschiene, hängen und daran befestigen. Die Aufhängevorrichtung 3, 4, 5 trägt ferner einen winkelförmigen Bügel 8, dessen vertikaler Schenkel die restliche Aufhängevorrichtung 3, 4, 5, nach oben überragt und dessen horizontaler Schenkel zum Drainagebehälter 1 zugewandt ist. Vertikal in diesem Text bezieht sich auf die bestimmungsgemässe Gebrauchslage der Vorrichtung.

Am freien Ende des horizontalen Schenkels ist eine Umlenkrolle 80 und zwei diese flankierende Führungsstifte 81 befestigt. Zwischen Umlenkrolle 80 und den Führungsstiften 81 wird der Drainageschlauch gehalten, ohne zusammengepresst zu werden. Der Bügel 8 bildet somit eine Zugentlastung und trägt das Gewicht des von der Umlenkrolle 80 zum Patienten führenden Drainageschlauchs 2.

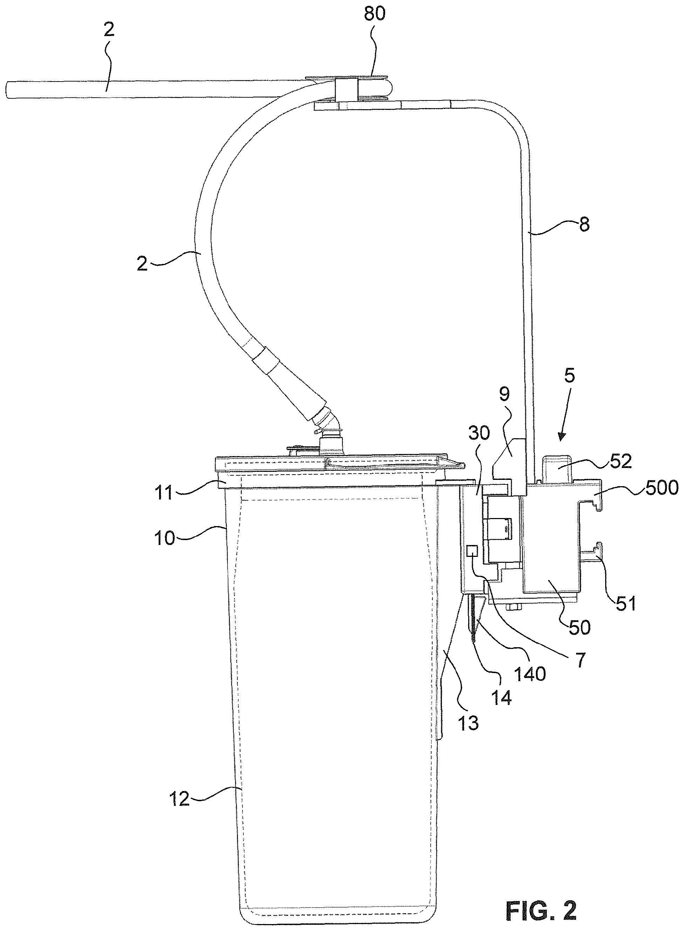

Wie in Figur 2 gut erkennbar ist, weist der Aussenbehälter 10 einen seitlich vorstehenden Befestigungsvorsatz 13 mit einem Nutstein auf. Am Nutstein ist ein Federelement 14 mit einer Rückhaltenase 140 angeordnet, wobei das Federelement eine nach unten gerichtete Verlängerung des vertikal verlaufenden Nutsteins bildet. Auch dies entspricht dem Behälter gemäss WO 2007/085100.

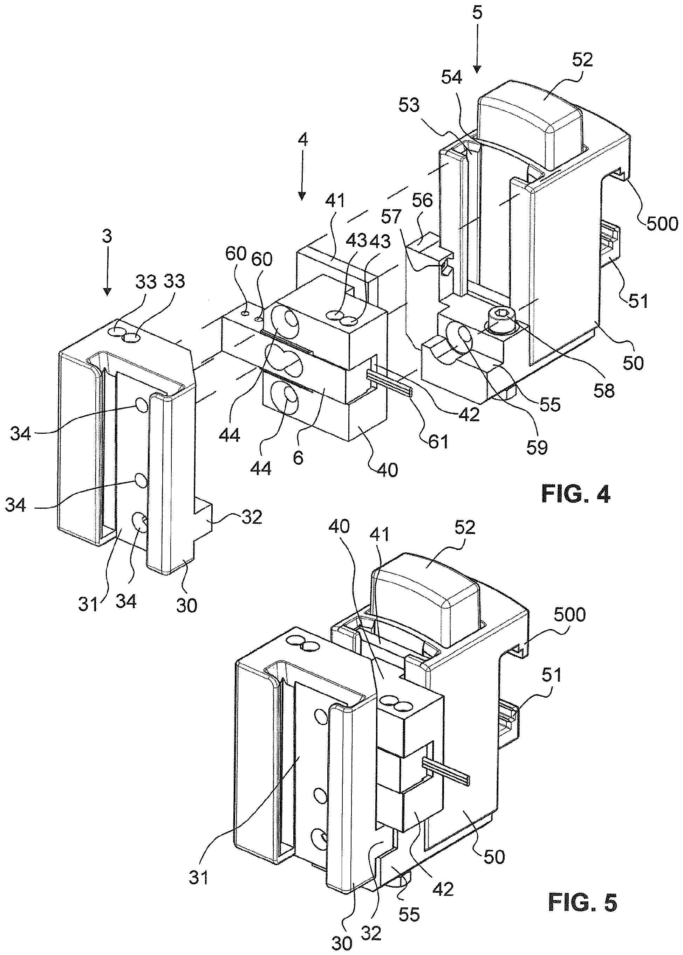

Dieser Nutstein ist in einer ersten Nut 31 (Figur 4) eines behälterseitigen Befestigungsteils 3 der erfindungsgemässen Aufhängevorrichtung eingeschoben und mit der federbelasteten Rückhaltenase 140 in seiner Position fixiert. Dadurch hängt der Behälter 1 an diesem ersten behälterseitigen Befestigungsteil 3. Die erste Nut 31 bildet in diesem Beispiel eine erste Befestigungseinheit.

Die Aufhängevorrichtung weist ferner ein zweites, schienenseitiges Befestigungsteil 5 auf. Dieses weist einen im Wesentlichen quaderförmigen Grundkörper 50 auf, an welchem eine obere feste Klaue 500 angeformt ist. Die Klaue 500 befindet sich auf der dem ersten Befestigungsteil 3 abgewandten Seite. Eine im Grundkörper 50 bewegbar gehaltene Klaue 51 bildet ein Gegenstück zur festen Klaue 500. Die bewegbare Klaue 51 ist mittels eines federbelasteten Druckknopfes 52, welcher aus einer Oberseite des Grundkörpers 50 herausragt, betätigbar. Mittels dieser zweiten Befestigungseinheit, nämlich den zwei Klauen 500, 51, lässt sich der zweite Befestigungsteil 5 und somit die restliche Aufhängevorrichtung wie auch der Drainagebehälter 1 an der Schiene S befestigen.

Zur Messung der Füllmenge im Beutel 12, insbesondere zur Bestimmung des Gewichts, weist die Aufhängevorrichtung zwischen dem ersten und dem zweiten Befestigungsteil 3, 5 ein Wägeteil 4 auf. Eine Anzeige 9 zeigt die gemessene Füllmenge, hier in Milliliter (ml). Andere Arten von Angaben, wie z.B. Gewichtsangaben oder graphische Anzeigen eines Füllstandes oder eines Füllverlaufes, sind auch möglich.

Im Folgenden sind anhand der Figuren 4 und 5 die einzelnen Teile der erfindungsgemässen Aufhängevorrichtung detaillierter beschrieben.

Das erste, behälterseitige Befestigungsteil 3 weist einen im Wesentlichen quaderförmigen Grundkörper 30 auf. Seine dem Behälter 1 zugewandte Seite ist mit der bereits erwähnten vertikal verlaufenden ersten Nut 31 versehen. Am Boden der ersten Nut 31 sind Durchgangslöcher 34 vorhanden, welche einen Zugang zu Befestigungsschrauben im nachfolgenden Wägeteil 4 ermöglichen. Auf der oberen Seite des Grundkörpers 30 ist mindestens ein Befestigungsloch, hier sind zwei Befestigungslöcher 33 vorhanden. Sie befinden sich in einem Randbereich der oberen Seite und dienen zur festen Verbindung mit einem Wägeelement 6, hier einer Wägezelle. Diese weist hierfür entsprechende Befestigungslöcher 60 auf. Vorzugsweise wird das behälterseitige Befestigungsteil 3 mit der Wägezelle 6 verschraubt. Andere Befestigungsarten sind jedoch auch möglich.

Auf der dem Behälter 1 abgewandten Seite des Grundkörpers 30 weist das behälterseitige Befestigungsteil 3 in seinem unteren Bereich einen ersten Vorsprung 32 auf, welcher dem zweiten, schienenseitigen Befestigungsteil 5 zugewandt ist. Die genannte abwandte Seite des Grundkörpers 30 ist ansonsten vorzugsweise plan und in Gebrauchslage in vertikaler Richtung verlaufend ausgebildet.

Das Wägeteil 4 weist ebenfalls einen im Wesentlichen quaderförmigen Grundkörper 40 mit einer zweiten Nut 42 auf, welche in horizontaler Richtung verläuft. In dieser zweiten Nut 42 ist die Wägezelle 6 gehalten, wobei die Wägezelle 6 der zweiten Nut 42 mindestens an einem Ende vorsteht und an dieser Seite mit dem behälterseitigen Befestigungsteil 3 verbunden ist. An ihrem anderen Ende ist die Wägezelle 6 mit dem Grundkörper 40 des Wägeteils 4 fest verbunden. Der Grundkörper 40 weist hierfür auf seiner Oberseite, vorzugsweise in einem Randbereich, Befestigungslöcher 43 auf. Auch hier erfolgt die feste Verbindung zwischen Grundkörper 40 und Wägezelle 6 vorzugsweise durch Verschrauben, wobei auch hier andere Arten von Befestigungen möglich sind.

Als Wägezelle 6 lassen sich bekannte Mittel einsetzen. Es handelt sich hierbei üblicherweise um Messmittel mit Dehnungsmessstreifen. Die Bezugsziffer 61 bezeichnet die Kabel, welche von der Wägezelle 6 in eine nicht dargestellte Elektronik und gegebenenfalls zu einer elektrischen Energiequelle führen. Es ist auch möglich, die gesamte oder einen Teil der Elektronik im Wägeteil 4 anzuordnen. In bevorzugten Ausführungsformen erfolgt die Datenübermittlung nicht nur an einen Display, sondern auch an externe Empfänger. In anderen Ausführungsformen fehlt ein Display. Des Weiteren ist es möglich, das Wägeteil mit einem akustischen Signalgeber auszustatten, welcher nach Massgabe des gemessenen Füllwertes aktivierbar ist. Anstelle der Wägezelle 6 kann auch ein Federelement mit Wegmessung, ein Piezosensor oder ein Drucksensor mit Linearführung eingesetzt werden.

An der der Nut 42 abgewandten Seite des Grundkörpers 40 ist ein in vertikaler Richtung verlaufender Nutstein 41 vorhanden. Vorzugsweise ist er einstückig am Grundkörper 40 angeformt. Dieser Nutstein 41 lässt sich in Eingriff mit einer dritten Nut 53 bringen, welche im Grundkörper 50 des zweiten, schienenseitigen Befestigungsteils 5 angeformt ist. Diese dritte Nut 53 endet an ihrer unteren Seite in einer doppelten Stufe, wobei dem oberen Stufenabsatz ein federbelasteter Bolzen 58 vorsteht, welcher in vertikaler Richtung entgegen der Federkraft verschiebbar ist. Auf diesem Bolzen 58 liegt das Wägeteil 4 auf. Die Federkraft muss dabei so gross gewählt sein, dass der Bolzen 58 und somit die Auflagefläche des Wägeteils 4 bei einer normalen Belastung, d.h. bei normaler Befüllung des Drainagebehälters, nicht nach unten gedrückt wird.

Am seitlichen Ende dieses oberen Stufenabsatzes, welches dem Bolzen 58 gegenüberliegt, ist eine seitliche Wand 56 hochgezogen. Vorzugsweise ist sie zusammen mit den Stufen einstückig ausgebildet. Sie weist ein durchgehendes Gewindeloch 57 auf. Eine durch dieses Gewindeloch eingedrehte Schraube, welche hier nicht dargestellt ist, greift in eine seitliche, vertikale Nut des Wägeteils 4 ein und fixiert so das Wägeteil 4 am schienenseitigen Befestigungsteil 5. Dabei ist das Wägeteil 4 in vertikaler Richtung entlang der Nut/Nutsteinverbindung nach wie vor verschiebbar. Ist keine Schraube eingeführt, so ist das Wägeteil 4 auch im Gebrauch von Hand ohne Werkzeuge vom schienenseitigen Befestigungsteil 5 lösbar.

Ist die doppelte Stufe ein separates Bauteil, so lässt sie sich beispielsweise mittels einer Schraubverbindung mit dem Grundkörper 50 des schienenseitigen Befestigungsteils 5 verbinden. Das entsprechende Gewindeloch 59 ist in Figur 4 gut erkennbar. Diese Variante hat den Vorteil, dass als Grundkörper 50 des schienenseitigen Befestigungsteils 5 die bekannten Aufhängevorrichtungen verwendet werden können. Es ist jedoch auch möglich, den Grundkörper 50 zusammen mit der doppelten Stufe und der Seitenwand 56 einstückig auszubilden.

Der untere Stufenabsatz des zweiten, schienenseitigen Befestigungsteils 5 bildet einen zweiten Vorsprung 55, welcher sich im zusammen gefügten Zustand der einzelnen Teile bis zum ersten, behälterseitigen Befestigungsteils 3 erstreckt und beabstandet unterhalb des ersten Vorsprungs 32 des behälterseitigen Befestigungsteils 3 zu liegen kommt. Der Abstand wird durch den Bolzen 58 bestimmt.

Die zwei Befestigungsteile 3, 5 und das Wägeteil 4 sind vorzugsweise im Wesentlichen aus Kunststoff gefertigt, vorzugsweise im Spritzgussverfahren.

Im bestimmungsgemässen Gebrauch ist die Aufhängevorrichtung zusammen gefügt. Das schienenseitige Befestigungsteil 5 wird an die Schiene S geklemmt und der Behälter 1 wird an das behälterseitige Befestigungsteil 3 gehängt. Wird der Behälter, genauer der Beutel 12, mit abgesaugter Körperflüssigkeit gefüllt, so wird auch das behälterseitige Befestigungsteil 3 nach unten gedrückt. Der schienenseitige Befestigungsteil und das Wägeteil 4 bleiben davon jedoch unbeeinflusst. Dadurch wird die Wägezelle 6 gebogen und ihre Biegung ergibt einen Messwert für den Füllstand.

Bei einer Überbelastung des ersten, behälterseitigen Befestigungsteils 3 reicht die Federkraft des Bolzens 58 nicht mehr aus, um das Wägeteil 4 in Position zu halten. Dieses wird ebenfalls nach unten gedrückt, jedoch maximal bis der Vorsprung 32 des behälterseitigen Befestigungsteils 3 auf dem Vorsprung 55 des schienenseitigen Befestigungsteils 5 aufliegt.

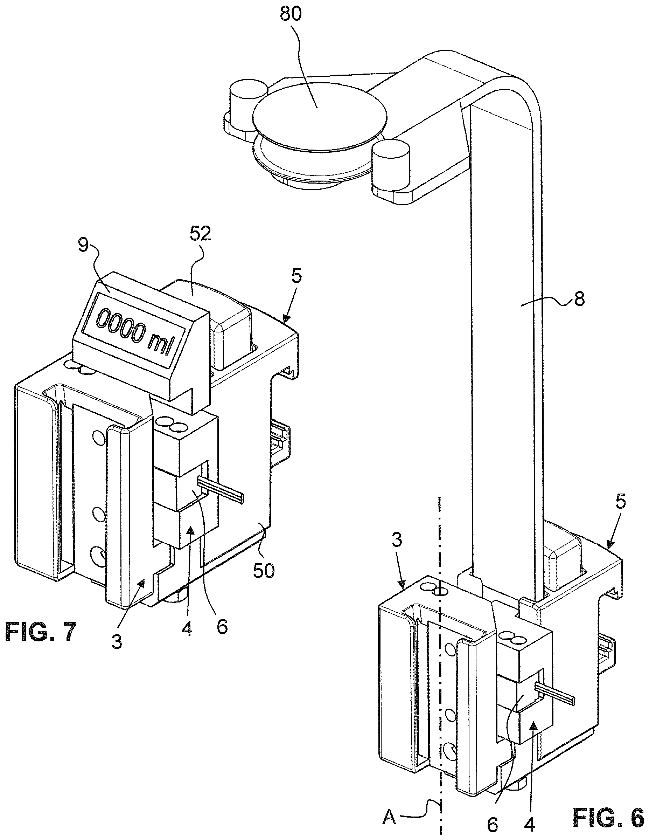

In Figur 6 ist erkennbar, wie der Bügel 8 der Zugentlastung in die Aufhängevorrichtung integriert ist. Er kann in einer einfachen Ausführungsform fest und im Gebrauch nicht lösbar mit dem Wägeteil 4 oder dem schienenseitigen Befestigungsteil 5 verbunden sein. Vorzugsweise ist jedoch hinter der dritten Nut 53 des schienenseitigen Befestigungsteils 5 eine ebenfalls vertikal verlaufende vierte Nut 54 vorhanden, in welche ein freies Ende des Bügels 8 eingeschoben und der Bügel 8 so gehalten werden kann. Eine Aufhängeachse A, welche durch die Aufhängevorrichtung definiert ist, ist ebenfalls dargestellt. Verläuft die Schiene S horizontal, so verläuft die Aufhängeachse A vertikal. Ansonsten bildet sie einen Winkel zur Vertikalen. Die oben beschriebenen Richtungen der einzelnen Teile beziehen sich auf den Fall, in welcher die Aufhängeachse A vertikal verläuft. Ansonsten sind die beschriebenen Richtungen parallel zur Aufhängeachse A zu verstehen.

In der Figur 7 ist erkennbar, dass auch der Display 9 aufgesetzt ist. Er kann an irgendeinem der beschriebenen Teile befestigt sein.

Ferner ist ein Neigesensor 7 vorhanden, welcher dazu dient, eine Schiefstellung des Behälters zu detektieren. Er kann ein drohendes Umkippen des Behälters feststellen und/oder eine Schiefstellung der Schiene detektieren, damit entsprechende Korrekturen bei der Messung eingebracht werden können. Der Neigesensor ist vorzugsweise möglichst nahe am Behälter angeordnet, beispielsweise im Grundkörper 30 des behälterseitigen Befestigungsteils 3. Er kann auch in die Elektronik integriert sein. Beispielsweise ist er ein Gyrosensor.

Die erfindungsgemässe Aufhängevorrichtung ermöglicht mit einfachen Mitteln eine Bestimmung einer Füllmenge eines Drainagebehälters, insbesondere eines Drainagebehälters mit Drainagebeutel.

### BEZUGSZEICHENLISTE

| | | | |
|---|---|---|---|
| 1 | Drainagebehälter | | |
| 10 | Aussenbehälter | 5 | schienenseitiges Befestigungsteil |
| 11 | Deckel | 50 | Grundkörper |
| 110 | Drainageanschluss | 500 | feste Klaue |
| 111 | Filterabdeckung | 51 | bewegbare Klaue |
| 112 | serieller Anschluss | 52 | Druckknopf |
| 113 | Befestigungsstutzen | 53 | dritte Nut |
| 114 | Handgriff | 54 | vierte Nut |
| 12 | Beutel | 55 | zweiter Vorsprung |
| 13 | Befestigungsvorsatz mit Nutstein | 56 | seitliche Wand |
| 14 | Federelement | 57 | Gewindeloch |
| 140 | Rückhaltenase | 58 | federbelasteter Bolzen |
| | | 59 | Durchgangsloch |
| 2 | Drainageschlauch | | |
| | | 6 | Wägeelement |
| 3 | behälterseitiges Befestigungsteil | 60 | Befestigungsloch |
| 30 | Grundkörper | 61 | Kabel |
| 31 | erste Nut | | |
| 32 | erster Vorsprung | 7 | Neigesensor |
| 33 | erstes Befestigungsloch | | |
| 34 | Durchgangslöcher | 8 | Bügel |
| | | 80 | Umlenkrolle |
| | | 81 | Führungsstift |
| 4 | Wägeteil | | |
| 40 | Grundkörper | 9 | Display |
| 41 | Nutstein | | |
| 42 | zweite Nut | A | Aufhängeachse |
| 43 | zweites Befestigungsloch | S | Schiene |
| 44 | Durchgangsloch | | |

## Patentansprüche

1. Aufhängevorrichtung eines Drainagebehälters (1), welcher zum Auffangen von abgesaugter Körperflüssigkeit dient, wobei die Aufhängevorrichtung auf einer ersten Seite eine erste Befestigungseinheit (31) zum Anhängen und Befestigen des Drainagebehälters (1) an die Aufhängevorrichtung aufweist und wobei die Aufhängevorrichtung auf einer zweiten Seite eine zweite Befestigungseinheit (500, 51) zum Anhängen und Befestigen der Aufhängevorrichtung an eine Schiene aufweist, wobei die erste Befestigungseinheit (31) in einem ersten Befestigungsteil (3) und die zweite Befestigungseinheit (500, 51) in einem zweiten Befestigungsteil (5) angeordnet ist, wobei die Aufhängevorrichtung ein Wägeteil (4) mit einem Wägeelement (6) umfasst, wobei das Wägeteil (4) mit dem ersten Befestigungsteil (3) und dem zweiten Befestigungsteil (5) verbunden ist oder verbindbar ist, um mit dem Wägeelement (6) eine relative Verschiebung zwischen dem ersten und dem zweiten Befestigungsteil (3, 5) zu messen, so dass eine Füllmenge der abgesaugten Körperflüssigkeit im Drainagebehälter (1) bestimmbar ist, und wobei die Aufhängevorrichtung eine Aufhängeachse (A) zur Aufhängung des Drainagebehälters (1) definiert, **dadurch gekennzeichnet, dass** die Aufhängevorrichtung einen Neigungssensor aufweist zur Feststellung eines Winkels zwischen der Aufhängeachse (A) und einer Vertikalen im Raum, in welchem sich die Aufhängevorrichtung befindet.

2. Aufhängevorrichtung nach Anspruch 1, wobei die erste Befestigungseinheit (31) zur Erstellung einer bei bestimmungsgemässem Gebrauch der Aufhängevorrichtung lösbaren Verbindung mit dem Drainagebehälter (1) ausgebildet ist und/oder wobei die zweite Befestigungseinheit (500, 51) zur Erstellung einer bei bestimmungsgemässem Gebrauch der Aufhängevorrichtung lösbaren Verbindung mit der Schiene ausgebildet ist.

3. Aufhängevorrichtung nach einem der Ansprüche 1 bis 2, wobei das erste Befestigungsteil (3) und das Wägeteil (4) eine fest miteinander verbundene und bei bestimmungsgemässem Gebrauch der Aufhängevorrichtung nicht zur Trennung voneinander vorgesehene Einheit bilden.

4. Aufhängevorrichtung nach Anspruch 3, wobei das zweite Befestigungsteil (5) eine bei bestimmungsgemässem Gebrauch der Aufhängevorrichtung lösbare Verbindung mit dem Wägeteil (4) bildet.

5. Aufhängevorrichtung nach Anspruch 3, wobei das zweite Befestigungsteil (5) und das Wägeteil (4) eine fest miteinander verbundene und bei Gebrauch nicht zur Trennung voneinander vorgesehene Einheit bilden, wodurch das erste Befestigungsteil (3), das Wägeteil (4) und das zweite Befestigungsteil (5) eine bei Gebrauch nicht zur Trennung voneinander vorgesehene Einheit bilden.

6. Aufhängevorrichtung nach einem der Ansprüche 1 bis 5, wobei das Wägeelement (6) einen ersten Bereich aufweist, welcher fest mit dem ersten Befestigungsteil (3) verbunden ist, und wobei das Wägeelement (6) einen zweiten Bereich aufweist, welcher fest mit dem Wägeteil (4) verbunden ist.

7. Aufhängevorrichtung nach einem der Ansprüche 1 bis 6, wobei das zweite Befestigungsteil (5) einen Vorsprung (55) aufweist, welcher sich bis zum ersten Befestigungsteil (3) erstreckt und welcher bei einer Gewichtsüberlast zur Auflage des ersten Befestigungsteils (3) dient.

8. Aufhängevorrichtung nach einem der Ansprüche 1 bis 7, wobei sie ferner eine Zugentlastung (8) für einen mit dem Drainagebehälter (1) verbundenen Drainageschlauch (2) zum Absaugen der Körperflüssigkeit aufweist.

9. Aufhängevorrichtung nach Anspruch 8, wobei die Zugentlastung einen Bügel (8) aufweist, welcher vom Wägeteil (4) und/oder vom zweiten Befestigungsteil (5) getragen ist.

10. Aufhängevorrichtung nach einem der Ansprüche 1 bis 9, wobei sie ferner einen Display (9) zur Anzeige der bestimmten Füllmenge aufweist.

11. Aufhängevorrichtung nach einem der Ansprüche 1 bis 10, wobei sie ferner eine Elektronikeinheit zur Übertragung der bestimmten Füllmenge an einen externen Empfänger aufweist.

12. Aufhängevorrichtung gemäss einem der Ansprüche 1 bis 11, wobei das zweite Befestigungsteil (5) einen Gewichtsüberlastschutz aufweist, welcher durch einen verschiebbaren federbelasteten Bolzen (58) zum Tragen des Wägeteils (4) gebildet ist.

13. Befestigungseinrichtung einer Aufhängevorrichtung eines Drainagebehälters (1), welcher zum Auffangen von abgesaugter Körperflüssigkeit dient, aufweisend ein erstes Befestigungsteil (3) mit einer ersten Befestigungseinheit (31) zum Anhängen und Befestigen des Drainagebehälters (1) an die Aufhängevorrichtung, sowie aufweisend ein Wägeteil (4) mit einem Wägeelement (6), wobei das Wägeteil (4) mit dem ersten Befestigungsteil (3) verbunden ist, und wobei das Wägeteil (4) mit einem zweiten Befestigungsteil (5) zwecks Verbindung mit einer Schiene verbindbar ist, um mit dem Wägeelement (6) eine relative Verschiebung zwischen dem ersten und zweiten Befestigungsteil (3, 5) zu messen, so dass eine Füllmenge der abgesaugten Körperflüssigkeit im Drainagebehälter (1) bestimmbar ist, **dadurch gekennzeichnet, dass** die Befestigungseinrichtung einen Neigungssensor (7) aufweist, um eine Schiefstellung des Drainagebehälters (1) und/oder der Schiene zu detektieren.

14. Drainagesystem mit einem Drainagebehälter und mit einer Aufhängevorrichtung nach einem der Ansprüche 1 bis 12, wobei der Drainagebehälter (1) einen Aussenbehälter (10), einen Beutel (12) zur Aufnahme im Aussenbehälter (10) und einen den Aussenbehälter (10) dicht verschliessenden Deckel (11) aufweist, wobei der Beutel (12) dicht mit dem Deckel (11) verbunden ist und gemeinsam mit dem Deckel (11) vom Aussenbehälter (10) entfernbar ist und wobei der Aussenbehälter (10) ferner eine Befestigungseinheit (13) zur Verbindung mit der ersten Befestigungseinheit (31) des ersten Befestigungsteils (3) aufweist.

## Claims

1. Suspension device for a drainage container (1) that is used to collect aspirated body fluid, wherein the suspension device has, on a first side, a first fastening unit (31) for mounting and fastening the drainage container (1) on the suspension device, and wherein the suspension device has, on a second side, a second fastening unit (500, 51) for mounting and fastening the suspension device on a rail, wherein the first fastening unit (31) is arranged in a first fastening part (3), and the second fastening unit (500, 51) is arranged in a second fastening part (5), wherein the suspension device comprises a weighing part (4) with a weighing element (6), wherein the weighing part (4) is connected or can be connected to the first fastening part (3) and to the second fastening part (5) so as to measure, with the weighing element (6), a relative displacement between the first fastening part (3) and the second fastening part (5), such that a filling quantity of the aspirated body fluid in the drainage container (1) can be determined, and wherein the suspension device defines a suspension axis (A) for the suspension of the drainage container (1), **characterized in that** the suspension device has a tilt sensor for determining an angle between the suspension axis (A) and a vertical within the room in which the suspension device is located.

2. Suspension device according to Claim 1, wherein the first fastening unit (31) is designed to produce a releasable connection to the drainage container (1) in the intended use of the suspension device, and/or wherein the second fastening unit (500, 51) is designed to produce a releasable connection to the rail in the intended use of the suspension device.

3. Suspension device according to either of Claims 1 and 2, wherein the first fastening part (3) and the weighing part (4) form a rigidly interconnected unit and are not designed to be separated from each other in the intended use of the suspension device.

4. Suspension device according to Claim 3, wherein the second fastening part (5) forms a releasable connection to the weighing part (4) in the intended use of the suspension device.

5. Suspension device according to Claim 3, wherein the second fastening part (5) and the weighing part (4) form a rigidly interconnected unit and are not designed to be separated from each other during use, as a result of which the first fastening part (3), the weighing part (4) and the second fastening part (5) form a unit that is not designed to be separated during use.

6. Suspension device according to one of Claims 1 to 5, wherein the weighing element (6) has a first area which is rigidly connected to the first fastening part (3), and wherein the weighing element (6) has a second area which is rigidly connected to the weighing part (4).

7. Suspension device according to one of Claims 1 to 6, wherein the second fastening part (5) has a projection (55) which extends as far as the first fastening part (3) and which serves to support the first fastening part (3) in the event of a weight overload.

8. Suspension device according to one of Claims 1 to 7, wherein it moreover has a strain relief means (8) for a drainage hose (2) which is connected to the drainage container (1) and which is used to aspirate the body fluid.

9. Suspension device according to Claim 8, wherein the strain relief means has a bracket (8) which is supported by the weighing part (4) and/or by the second fastening part (5).

10. Suspension device according to one of Claims 1 to 9, wherein it moreover has a display (9) for showing the determined filling quantity.

11. Suspension device according to one of Claims 1 to 10, wherein it moreover has an electronics unit for transmitting the determined filling quantity to an external receiver.

12. Suspension device according to one of Claims 1 to 11, wherein the second fastening part (5) has a weight overload protection which is formed by a displaceable spring-loaded bolt (58) for supporting the weighing part (4).

13. Fastening device of a suspension device of a drainage container (1) that is used to collect aspirated body fluid, comprising a first fastening part (3) with a first fastening unit (31) for mounting and fastening the drainage container (1) on the suspension device, and comprising a weighing part (4) with a weighing element (6), wherein the weighing part (4) is connected to the first fastening part (3), and wherein the weighing part (4) can be connected to a second fastening part (5) for the purpose of connection to a rail, so as to measure, with the weighing element (6), a relative displacement between the first fastening part (3) and the second fastening part (5) such that a filling quantity of the aspirated body fluid in the drainage container (1) can be determined, **characterized in that** the fastening device has a tilt sensor, in order to detect an inclined position of the drainage container (1) and/or of the rail.

14. Drainage system with a drainage container (1) and with a suspension device according to one of Claims 1 to 12, wherein the drainage container (1) has an outer container (10), a bag (12) to be received in the outer container (10), and a lid (11) tightly closing the outer container (10), wherein the bag (12) is connected sealingly to the lid (11) and can be removed together with the lid (11) from the outer container (10), and wherein the outer container (10) moreover has a fastening unit (13) for connecting to the first fastening unit (31) of the first fastening part (3).

## Revendications

1. Dispositif de suspension d'un récipient de drainage (1), lequel sert à recueillir du liquide corporel aspiré, où le dispositif de suspension présente sur un premier côté une première unité de fixation (31) permettant d'accrocher et de fixer le récipient de drainage (1) au dispositif de suspension et où le dispositif de suspension présente sur un deuxième côté une deuxième unité de fixation (500, 51) permettant d'accrocher et de fixer le dispositif de suspension à un rail, où la première unité de fixation (31) est agencée dans une première partie de fixation (3) et la deuxième unité de fixation (500, 51) est agencée dans une deuxième partie de fixation (5), où le dispositif de suspension comporte une partie de pesée (4) munie d'un élément de pesée (6), où la partie de pesée (4) est reliée ou peut être reliée à la première partie de fixation (3) et à la deuxième partie de fixation (5) pour mesurer au moyen de l'élément de pesée (6) un déplacement relatif entre la première et la deuxième partie de fixation (3, 5), de sorte qu'une quantité de remplissage du liquide corporel aspiré dans le récipient de drainage (1) peut être déterminée, et où le dispositif de suspension définit un axe de suspension (A) pour la suspension du récipient de drainage (1), **caractérisé en ce que** le dispositif de suspension présente un détecteur d'inclinaison pour déterminer un angle entre l'axe de suspension (A) et une verticale dans l'espace, dans lequel se situe le dispositif de suspension.

2. Dispositif de suspension selon la revendication 1, où la première unité de fixation (31) est réalisée pour établir une liaison amovible lors d'une utilisation normale du dispositif de suspension avec le récipient de drainage (1) et / ou où la deuxième unité de fixation (500, 51) est réalisée pour former une liaison amovible lors de l'utilisation normale du dispositif de suspension avec le rail.

3. Dispositif de suspension selon une des revendications 1 à 2, où la première partie de fixation (3) et la partie de pesée (4) forment une unité étant reliée de manière fixe et non destiné à la séparation lors de l'utilisation normale du dispositif de suspension.

4. Dispositif de suspension selon la revendication 3, où la deuxième partie de fixation (5) forme une liaison amovible avec la partie de pesée (4) lors de l'utilisation normale du dispositif de suspension.

5. Dispositif de suspension selon la revendication 3, où la deuxième partie de fixation (5) et la partie de pesée (4) forment une unité reliée de manière fixe et non destiné à la séparation lors de l'utilisation, selon quoi la première partie de fixation (3), la partie de pesée (4) et la deuxième partie de fixation (5) forment une unité non destinée à la séparation lors du l'utilisation.

6. Dispositif de suspension selon une des revendications 1 à 5, où l'élément de pesée (6) présente un premier domaine, lequel est relié de manière fixe à la première partie de fixation (3), et où l'élément de pesée (6) présente un deuxième domaine, lequel est relié de manière fixe à la partie de pesée (4).

7. Dispositif de suspension selon une des revendications 1 à 6, où la deuxième partie de fixation (5) présente une saillie (55) laquelle s'étend jusqu'à la première partie de fixation (3) et laquelle sert de surface de repos de la première partie de fixation (3) lors d'une surcharge de poids.

8. Dispositif de liaison selon une des revendications 1 à 7, où en outre celui-ci présente une décharge de traction (8) pour un tuyau de drainage (2) relié au récipient de drainage (1) pour l'aspiration du fluide corporel.

9. Dispositif de suspension selon la revendication 8, où la décharge de traction présent un étrier (8) lequel est supporté par la partie de pesée (4) et / ou par la deuxième partie de fixation (5).

10. Dispositif de suspension selon une des revendications 1 à 9, où celui-ci présente en outre un écran (9) pour l'affichage d'une certaine quantité de remplissage.

11. Dispositif de suspension selon une des revendications 1 à 10, où celui-ci présente en outre une unité électronique pour la transmission de la quantité de remplissage déterminée à un destinataire externe.

12. Dispositif de suspension selon une des revendications 1 à 11, où la deuxième partie de fixation (5) présente une protection contre une surcharge de poids laquelle est formée par un boulon (58) prétensioné pouvant être déplacé pour supporter la partie de pesée (4).

13. Installation de fixation d'un dispositif de suspension d'un récipient de drainage (1) lequel sert à recueillir un liquide corporel aspiré, présentant une première partie de fixation (3) muni d'une première unité de fixation (31) permettant d'accrocher et de fixer le récipient de drainage (1) au dispositif de suspension, ainsi que présentant une partie de pesée (4) munie d'un élément de pesée (6), où la partie de pesée (4) est reliée à une première partie de fixation (3), et où la partie de pesée (4) peut être reliée à une deuxième partie de fixation (5) pour une liaison à un rail, afin de mesurer au moyen de l'élément de pesée (6) un déplacement relatif entre la première et deuxième partie de fixation (3, 5), de sorte qu'une quantité de remplissage du liquide corporel aspiré dans le récipient de drainage (1) peut être déterminée, **caractérisée en ce que** l'installation de fixation présente un détecteur d'inclinaison (7) pour déterminer une position oblique du récipient de drainage (1) et / ou du rail.

14. Système de drainage muni d'un récipient de drainage (1) et d'un dispositif de suspension selon une des revendications 1 à 12, où le dispositif de drainage (1) présente un récipient extérieur (10), un poche (12) pour la réception dans le récipient extérieur (10) et un couvercle (11) fermant de manière étanche le récipient extérieur (10), où la poche (12) est reliée de manière étanche avec le couvercle (11) et peut être enlevée du récipient extérieur (10) ensemble avec le couvercle (11), et où le récipient extérieur (10) présente en outre une unité de fixation (13) pour une liaison à la première unité de fixation (31) de la première partie de fixation (3).
